# EUROPEAN PATENT APPLICATION

(11) **EP 3 862 043 A2**
(43) Date of publication of application: **11.08.2021**
(21) Application number: 20217223.5
(22) Date of filing: 24.12.2020
(51) Int. Cl.: A61M 25/00

(54) **A CATHETER**

(30) Priority: 10.01.2020 AU 2020100056
(71) Applicant: Van der Weegen, Clemens M., Newington, NSW 2127 (AU)
(72) Inventor: Van der Weegen, Clemens M., Newington, NSW 2127 (AU)
(74) Representative: Marles, Alan David

(57) **Abstract**

A catheter (10) adapted to be located at least partially in a patient's urethra (15), the catheter (10) comprising: an elongate tube (20) having a proximal end portion (25) and a distal end portion (30); the distal end portion (30) being provided with a distal retention member (35) to retain the distal end portion (30) within the patient's bladder (40) during use; a length of the elongate tube (20) adjacent the proximal end portion (30) being provided with a proximal retention member (65) to retain the elongate tube (20) within the urethra; and the proximal retention member (65) including an enlarged section to be located within a cavity (90) that surrounds a portion of the patient's urethra (15) during use, with the cavity (90) being located adjacent an exposed end portion (95) of the patient's urethra (15).

## Description

### FIELD

The present invention relates to catheters, and more particularly, but not exclusively to, urinary catheters that facilitate drainage of urine from a patient's bladder, or for irrigation of the patient's bladder with fluids.

### BACKGROUND

Urinary catheters are a known medical device and are commonly used in hospital or home settings for patients that have difficulties when urinating and require assistance with emptying their bladder.

A disadvantage of known urinary catheters is that they are susceptible to build-up of bacteria, particularly when a catheter has been in place in a patient's urethra for an extended period of time. This build-up of bacteria may cause an infection of the patient's urinary tract. Bacteria commonly enters a catheter via the part that is exposed to the external environment that is also in contact with the patient's urethra. In a male patient, for example, this is the part of the catheter that is located at the entry point at the tip of the patient's penis during use. This entry point is typically where the bacteria from the external environment (for example, from the patient touching the catheter and transferring bacteria from their hands to the catheter) gathers, and spreads through the otherwise sterile urethra of the patient.

The above problem is exacerbated when the catheter moves around during use, for example, from movement of the patient, or from involuntary movement (for example, an involuntary erection) of the patient's penis. Movement of the catheter may cause the bacteria to spread along the length of the catheter, which may eventually result in the bacteria entering the bladder and other parts of the urinary tract, leading to unwanted urinary tract infections.

### SUMMARY

It is an object of the present invention to substantially overcome, or at least ameliorate, one or more of the disadvantages of existing arrangements, or at least provide a useful alternative to existing arrangements.

There is disclosed herein a catheter adapted to be located in a patient's urethra, the catheter comprising:
an elongate tube having a proximal end portion and a distal end portion;
the distal end portion being provided with a distal retention member to retain the distal end portion within the patient's bladder during use;
a length of the elongated tube adjacent the proximal end portion being provided with a proximal retention member to retain the proximal end portion within the urethra; and
the proximal retention member including an expandable section to be located within an existing natural cavity that surrounds a portion of the patient's urethra during use, with the existing natural cavity being located adjacent an exposed end portion of the patient's urethra.

Preferably, the proximal retention member includes an inflation port and the expandable section comprises a balloon having a flexible wall surrounding an interior chamber, with the inflation port being in fluid communication with the interior chamber of the balloon.

Preferably, the inflation port is operatively associated with a fluid pump to facilitate entry of fluid into the interior chamber of the balloon to expand the flexible wall, whereby in an expanded state, the flexible wall engages an inner wall of the existing natural cavity to seal the existing natural cavity, and thereby the urethra, from an external environment, and whereby the existing natural cavity is also sealed to prevent leakage.

Preferably, the fluid is saline solution provided as a predetermined amount from the fluid pump.

Preferably, the flexible wall engages the inner wall of the existing natural cavity to seal the existing natural cavity, thereby preventing bacteria from entering the urethra, and to also limit or prevent leakage of urine from the urethra.

There is also disclosed herein a retention member for use with a catheter that is adapted to be located in a patient's urethra, the retention member comprising:
an elongate tube having an inner wall that is slidably engageable with an outer wall of the catheter; and
an expandable section that is located within an existing natural cavity surrounding a portion of the patient's urethra during use, with the existing natural cavity being located adjacent an exposed end portion of the patient's urethra.

Preferably, the expandable section comprises a balloon having a flexible wall surrounding an interior chamber, and the retention member further comprises an inflation port that is in fluid communication with the interior chamber of the balloon to facilitate expansion of the flexible wall, whereby in an expanded state, the flexible wall engages an inner wall of the existing natural cavity to seal the existing natural cavity, and thereby the urethra, from an external environment.

### BRIEF DESCRIPTION OF THE DRAWINGS

Preferred forms of the present invention will now be described, by way of example only, with reference to the accompanying drawings, in which:
Figure 1 is a schematic front view of a catheter;
Figures 2 to 5 are schematic front views of a retention member in isolation;
Figures 6 and 7 are schematic views of various components associated with the retention member of Figures 2 to 5; and
Figure 8 is a schematic front view of a further embodiment of a catheter.

### DETAILED DESCRIPTION OF EMBODIMENTS

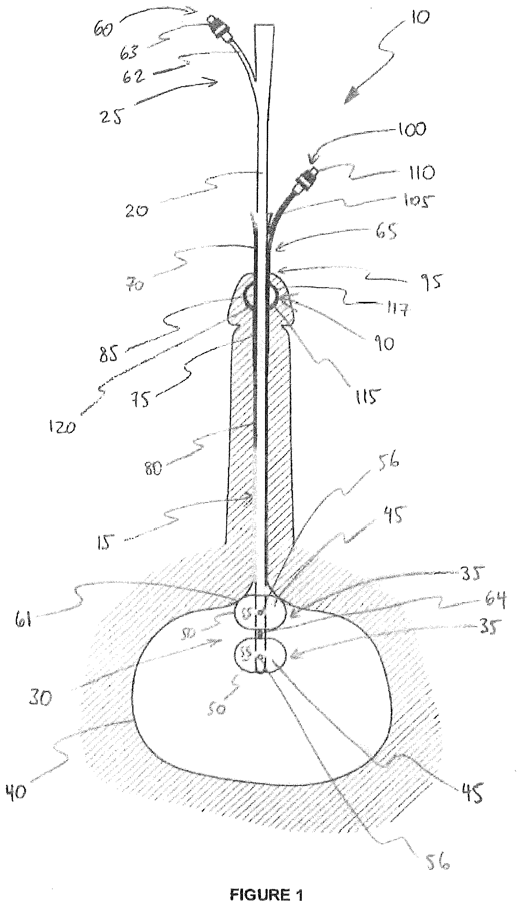

In Figure 1 of the accompanying drawings, there is schematically depicted a catheter 10 that is adapted to be located at least partially in a patient's urethra 15. The catheter 10 includes an elongate tube 20 having a proximal end portion 25 and a distal end portion 30. The elongate tube 20 may be formed from a flexible material such as silicone rubber, polyurethane, latex or the like.

The distal end portion 30 of the elongate tube 20 is provided with a distal retention member 35 that is adapted to retain the distal end portion 30 within the patient's bladder 40 during use. In the depicted embodiment, the catheter 10 is shown to be provided with two distal retention members 35. However, it will be understood that the catheter 10 may alternatively be provided with one, or more than two distal retention members 35, depending on the use and requirements of the patient.

The distal retention member 35 includes an expandable section comprising a balloon 45 having a flexible wall 50 surrounding an interior chamber 55, with the interior chamber 55 being in fluid communication with a fluid port 60 located at the proximal end portion 25 of the elongate tube 20. An opening 56 provides the passage between the interior chamber 55 and the interior of the elongate tube 20. It will be understood from the Figures that the flexible wall 50 of the balloon 45 may be arranged to engage an inner wall 61 of the bladder 40 that leads to the urethra 15, and is dimensioned to have a diameter that is larger than a diameter of the urethra, so as to prevent travel along the urethra 15 when the balloon 45 is expanded.

The fluid port 60 is provided by an elongate tube 62 and an end part 63 that facilitates connection to a pump (for example, a syringe), a drainage tube and/or a collection bag (not shown). A fluid pump, for example, may facilitate entry of air (or other fluid) into the interior chamber 55 of the balloon 45, via the elongate tube 20, to inflate the balloon 45 to an expanded state after insertion into the urethra 15.

The distal end portion 30 of the elongate tube 20 also includes an opening 64 through which fluid (for example, urine from the patient's bladder 40) may travel.

A length of the elongate tube 20 is provided with a proximal retention member 65 that is adapted to retain the elongate tube 20 within the urethra 15. It will be appreciated that the proximal retention member 65 may be located anywhere along the length of the elongate tube 20 between the proximal and distal end portions 25 and 30, depending on the use and requirements of the patient. In the embodiment depicted in Figure 1, the proximal retention member 65 is provided with an elongate tube 70 having an inner wall 75 that slidably engages with an outer wall 80 of the elongate tube 20. The elongate tube 70 may also be formed from a flexible material such as silicone rubber, polyurethane, latex or the like.

The proximal retention member 65 includes an enlarged section 85 that is locatable within an existing natural cavity 90 that surrounds a portion of the patient's urethra 15 during use. The existing natural cavity 90 is located adjacent an exposed end portion 95 of the patient's urethra 15, and forms part of the urethra 15. It will be understood by a person skilled in the art that the existing natural cavity 90 is a naturally occurring part of a penis and located within the head of the penis, towards the opening that leads to the urethra 15. In the depicted embodiment, the enlarged section 85 is locatable about halfway along the length of the elongate tube 70, however, it is envisaged that the enlarged section 85 may be locatable anywhere along the length of the elongate tube 70.

The proximal retention member 65 includes an inflation port 100 provided by an elongate tube 105 that extends from the elongate tube 70 and an end part 110 that facilitates connection to a pump.

The enlarged section 85 includes an expandable balloon 115 having a flexible wall 117 surrounding an interior chamber 118, which is in turn in fluid communication with the inflation port 100. The inflation port 100 may be operatively associated with a pump (not shown), such as a disposable syringe with a predetermined (measured) amount of fluid, to facilitate entry of the fluid into the interior chamber 118 of the balloon 115 to expand the flexible wall 117. In a preferred form, the pump facilitates entry of saline solution into the interior chamber 118 of the balloon 115 to inflate the balloon 115 to an expanded state. In the expanded state, the flexible wall of the balloon 115 engages an inner wall 120 of the existing natural cavity 90 so as to seal the existing natural cavity 90, and thereby the urethra 15, from an external environment. This sealing may also limit or prevent urine from leaking out of the urethra 15 into the external environment. It will be appreciated that in the expanded state, the balloon 115 is firmly engaged within the existing natural cavity 90 so as to minimise or prevent the movement of the catheter 10 along the urethra 15. It will be appreciated that once the balloon 115 is in place in the existing natural cavity 90, the distal retention member 35 is free to move within the bladder 40, with the distal-most retention member serving as a cushion or buffer to keep the opening 64 clear, i.e. to prevent the internal walls of the bladder 40 from being drawn into the opening 64. Accordingly, the distal retention member 35 does not necessarily need to serve as a mechanism to keep the catheter 20 locked inside the bladder 40, except in an emergency when someone attempts to forcibly remove the catheter 20 from the penis. It will further be appreciated that the balloon 115 may be any size and located at any location suitable to meet the particular penis size of the patient.

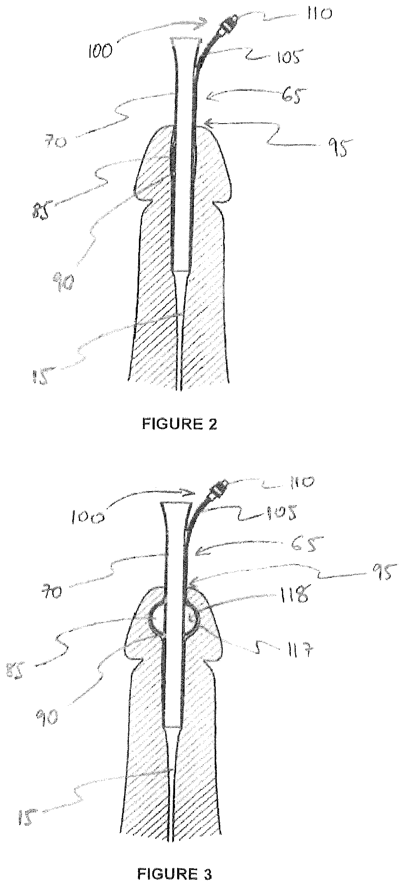

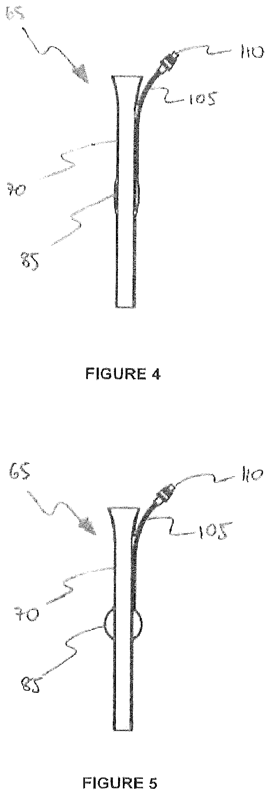

Figures 2 to 5 depict an embodiment of the proximal retention member 65 in isolation. Figures 2 and 4 show the balloon 115 in an unexpanded state in which the proximal retention member 65 is relatively free to travel along the urethra 15, whilst Figures 3 and 5 show the balloon 115 in an expanded state in which the balloon 115 is engaged within the existing natural cavity 90 so as to minimise or prevent the movement of the proximal retention member 65 along the urethra 15. It will be appreciated that by having the proximal retention member 65 as a separate, removable item, it is possible for different companies that manufacture catheters to use their existing catheters without having to alter their design, as the proximal retention member 65 in this embodiment may be used as an insert or sleeve. When used as an insert or sleeve, antibacterial gel may be applied to the proximal retention member 65 (for example, along the inner wall 75 of the elongate tube 70) such that the proximal retention member 65 may slide easily along the outer wall 80 of the elongate tube 20. It will thus be appreciated that with this arrangement, the practitioner (for example, a doctor or a nurse) may be able to remove and replace the rest of the catheter 10 (for example, the elongate tube 20 and the distal retention member 35) from the patient without necessarily having to remove the insert or sleeve.

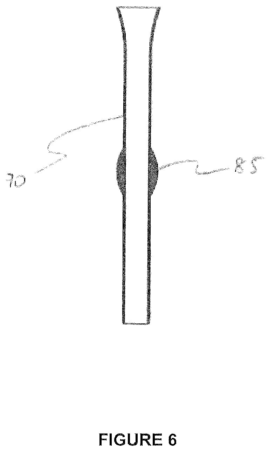

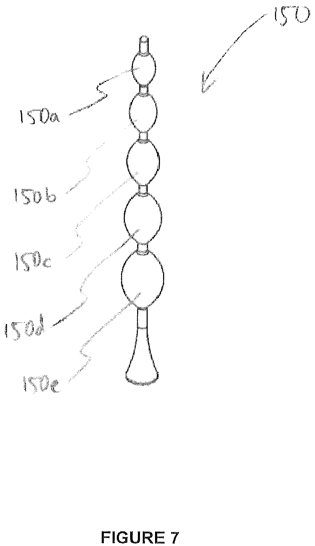

Figures 6 and 7 show various components associated with the proximal retention member 65 to facilitate the positioning of the proximal retention member 65 within the existing natural cavity 90. Figure 7, for example, shows a tool 150 having various enlarged portions 155a, 155b, 115c, 155d and 155e that correspond to various sizes of the existing natural cavity 90, to allow the practitioner to correctly gauge the size or volume of the patient's existing natural cavity 90. Once the size or volume of the patient's existing natural cavity 90 is determined, any subsequent measured amount of fluid corresponding to the next size in the tool 150 will be provided by the pump (syringe) so as to lock the catheter 20 in place. For example, if the size or volume of the existing natural cavity 90 corresponds to the size or volume of part 150a as measured using the tool 150, then the amount of fluid provided by the pump (syringe) will correspond to the size or volume of part 150b.

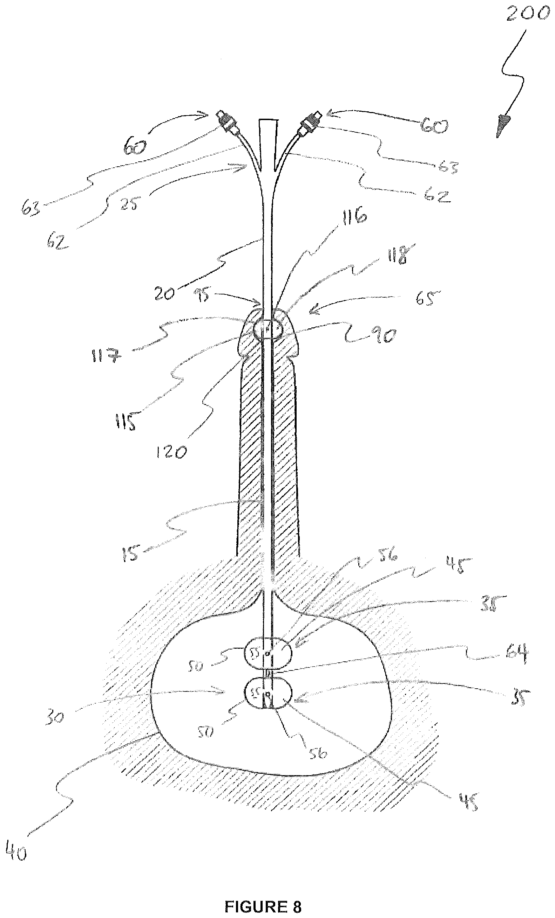

Figure 8 shows another embodiment of a catheter 200, which functions in a substantially similar manner to the catheter 10 described above, with like reference numerals being used to depict like features. In this embodiment, the proximal retention member 65 is provided by the balloon 115 only, and the interior chamber 118 of the balloon 115 is in fluid communication with the elongate tube 20 via an opening 116. This embodiment also includes two fluid ports 60 located at the proximal end portion 25 of the elongate tube 20, one of which is in fluid communication with the interior chamber 118 of the balloon 115, and the other of which is in fluid communication with the interior chamber 55 of the balloon 45. It will be understood that there are two internal lines within the elongate tube 20 that allow the two fluid ports 60 to being fluid communication with the two different chambers 118 and 55. Although the drawing shows both fluid ports 60 at about the same location, it will be appreciated that one of the fluid ports 60 may be about 2 to 3 cm lower than the other fluid port 60 along the elongate tube 20, so as to minimise confusion as to the amount of measured fluid that is to be introduced into each of the chambers 118 and 55. Accordingly, one of the fluid ports 60 located at the proximal end portion 25 of the elongate tube 20 may be used to introduce fluid into the interior chamber 118 of the balloon 115, thereby expanding the balloon 115 and securing the balloon 115 in place within the existing natural cavity 90. The practitioner would apply antibacterial gel or the like around the balloon 115 prior to inserting the balloon 115 into the existing natural cavity 90 (via the exposed end portion 95 and the urethra 15). The other one of the fluid ports 60 located at the proximal end portion 25 of the elongate tube 20 may be used to introduce fluid into the interior chamber 55 of the balloon 45, thereby expanding the balloon 55 within the bladder 40. It will thus be appreciated that two different amounts of fluids may be introduced into the two different chambers 118 and 55.

Various forms of the catheters and retention members described may therefore have the advantage of at least minimising or preventing movement of the catheter during use, thereby reducing the risk of infections from the external environment (particularly from longer term use). The expandable balloons of the proximal retention member may also at least seal the existing natural cavity, and thereby at least a section of the urethra, from an external environment, and may also at least limit or prevent leakage of urine from at least a section of the urethra to the external environment. The catheters and retention members may also be easily adapted according to a patient's specific requirements.

Although the invention has been described with reference to a preferred embodiment, it will be appreciated by those persons skilled in the art that the invention may be embodied in many other forms.

## Claims

1. A catheter adapted to be located at least partially in a patient's urethra, the catheter comprising:
an elongate tube having a proximal end portion and a distal end portion;
the distal end portion being provided with a distal retention member to retain the distal end portion within the patient's bladder during use;
a length of the elongate tube adjacent the proximal end portion being provided with a proximal retention member to retain the elongate tube within the urethra; and
the proximal retention member including an enlarged section to be located within an existing natural cavity that surrounds a portion of the patient's urethra during use, with the existing natural cavity being located adjacent an exposed end portion of the patient's urethra.

2. The catheter of claim 1, wherein the proximal retention member includes an inflation port and the enlarged section comprises an expandable balloon having a flexible wall surrounding an interior chamber, with the inflation port being in fluid communication with the interior chamber of the balloon.

3. The catheter of claim 2, wherein the inflation port is operatively associated with a fluid pump to facilitate entry of fluid into the interior chamber of the balloon to expand the flexible wall, whereby in an expanded state, the flexible wall engages an inner wall of the existing natural cavity to seal the existing natural cavity, and thereby at least a section of the urethra, from an external environment, so as to at least preventing bacteria from entering the urethra, and to also at least limit or prevent leakage of urine from the urethra.

4. The catheter of claim 3, wherein the fluid is saline solution provided as a predetermined amount from the fluid pump.

5. The catheter of claim 1, wherein the proximal retention member is provided by an expandable balloon having a flexible wall surrounding an interior chamber, the flexible wall being expandable to engage an inner wall of the existing natural cavity to seal the existing natural cavity, and thereby at least a section of the urethra, from an external environment, so as to at least preventing bacteria from entering the urethra, and to also at least limit or prevent leakage of urine from the urethra.
